Europäisches Patentamt

European Patent Office

Office européen des brevets

(19)

(11) **EP 0 818 530 B1**

(12) **FASCICULE DE BREVET EUROPEEN**

(45) Date de publication et mention
de la délivrance du brevet:
**10.08.2005 Bulletin 2005/32**

(51) Int Cl.[7]: **C12N 1/18**

(21) Numéro de dépôt: **97401615.6**

(22) Date de dépôt: **07.07.1997**

(54) **Souches de levure de fermentation brassicole**

Stämme der Biefhefe

Brewers yeast strains

(84) Etats contractants désignés:
**BE DE DK ES FR GB IE IT NL**

(30) Priorité: **08.07.1996 FR 9608467**

(43) Date de publication de la demande:
**14.01.1998 Bulletin 1998/03**

(73) Titulaire: **BRASSERIES KRONENBOURG**
**67200 Strasbourg (FR)**

(72) Inventeurs:
• **Gendre, François**
**67000 Strasbourg (FR)**
• **Brignon, Pierre**
**67200 Strasbourg (FR)**
• **Proth, Jacques**
**67450 Mundolsheim (FR)**
• **Shingleton, Myriam**
**67202 Wolfisheim (FR)**

(74) Mandataire: **Vialle-Presles, Marie José et al**
**Cabinet ORES,**
**36,rue de St Pétersbourg**
**75008 Paris (FR)**

(56) Documents cités:
**EP-A- 0 228 009      EP-A- 0 339 532**
**EP-A- 0 511 108**

• **DATABASE WPI Section Ch, Week 7515 Derwent**
**Publications Ltd., London, GB; Class D16, AN**
**75-25506W XP002028372 & SU 415 983 A**
**(GOLANT M B ET AL) , 15 novembre 1974**
• **DATABASE WPI Section Ch, Week 9614 Derwent**
**Publications Ltd., London, GB; Class D16, AN**
**96-138114 XP002028373 & RU 2 039 813 C (AS**
**USSR GEN GENETICS INST) , 20 juillet 1995**

**Description**

La présente Invention est relative à de nouvelles souches de levures de fermentation brassicole et un procédé de sélection de ces levures.

La fermentation brassicole comporte plusieurs étapes : la fermentation principale ou fermentation alcoolique qui dure en moyenne quelques jours, suivie d'une phase de maturation ou garde chaude, d'une durée de quelques jours supplémentaires (3 à 4 jours) et complétée par une phase de stabilisation ou garde froide qui dure de 4 à 15 jours et pendant laquelle la température est maintenue à 0°C.

La phase de maturation a principalement pour but de permettre la résorption par les levures des dicétones, telles en particulier que la 2,3-pentanedione et surtout le diacétyle, qui sont responsables de la formation d'arômes indésirables. Par exemple, au dessus d'une concentration seuil de 0,05 à 0,1 ppm, le diacétyle donne à la bière un goût de beurre rance.

Le diacétyle résulte de l'oxydation non-enzymatique de l'alpha-acétolactate, qui est un intermédiaire de la voie de biosynthèse de la valine.

L'alpha-acétolactate est produit pendant la fermentation, et résulte de la transformation du pyruvate par l'acétolactate synthase, produit du gène ILV2. Dans la voie normale de biosynthèse de la valine, l'alpha-acétolactate est ensuite transformé en dihydroxyisovalérate par la réductoisomérase, produit du gène ILV5.

Lorsque l'alpha-acétolactate est produit en excès, il s'accumule dans le milieu de culture, où il est oxydé en diacétyle. La levure est capable de dégrader le diacétyle libre en composés qui, aux concentrations rencontrées dans la bière sont sensoriellement neutres. Ces composés sont l'acétoïne et le 2,3-butanediol.

L'oxydation de l'alpha-acétolactate en diacétyle doit donc être effectuée avant l'élimination des levures, pour que celles-ci puissent le dégrader. L'un des rôles essentiels de la phase de maturation est de permettre cette oxydation, et la dégradation du diacétyle qui s'ensuit.

Pour résoudre les problèmes liés à la présence du diacétyle, en cours de fermentation brassicole et afin d'accélérer le procédé de fermentation, en racourcissant la phase de maturation deux approches ont été préconisées :

- accélérer la conversion de l'alpha-acétolactate en diacétyle ou,
- réduire la formation de l'alpha-acétolactate.

La publication de J. R. M. HAMMOND, parue dans YEAST, vol. 11, (1995) présente (pages 1619-1620) trois techniques différentes ressortant de l'une ou l'autre de ces approches, et permettant de réduire la quantité de diacétyle produit au cours de la fermentation.

a) Pour accélérer la conversion de l'alpha-acétolactate, il a été proposé d'utiliser l'alpha-acétolactate décarboxylase (ALDC), qui est une enzyme bactérienne transformant directement l'alpha-acétolactate en acétoïne.

Cette enzyme, qui n'est pas présente dans la levure, peut être ajoutée au milieu de fermentation ; ceci entraîne toutefois un surcoût de production important.

Le gène ALDC d'origine bactérienne peut également être cloné et exprimé dans la levure. Les levures ainsi obtenues constituent des organismes génétiquement modifiés, dont la mise en oeuvre est soumise à des contraintes réglementaires strictes.

Il a également été proposé d'augmenter le flux métabolique de la voie de biosynthèse de la valine en aval de l'alpha-acétolactate, en augmentant le nombre de copies du gène ILV5. Cependant, comme la précédente, cette technique implique la modification des levures par génie génétique, et comporte donc les mêmes inconvénients.

b) Dans le cadre de la deuxième approche, il a été proposé de sélectionner des mutants dans lesquels l'activité de l'acétolactate synthase, produit du gène ILV2, est réduite. Ces mutants sont obtenus sur la base de leur résistance à un herbicide, le sulphométuron méthyle ; certains des clones résistants, présentent une réduction de l'activité de l'acétolactate synthase, qui se traduit par une production moindre de l'alpha-acétolactate. Ces mutants sont sélectionnés sur la base de leur croissance lente sur milieu minimum en absence de valine et d'isoleucine.

Les Inventeurs ont maintenant obtenu par sélection à partir d'une préparation industrielle de ferment, constituée par un mélange de souches de *Saccharomyces cerevisiae,* des souches qui produisent de très faibles quantités des précurseurs du diacétyle et de la 2,3-pentanedione, et qui en outre résorbent très rapidement ces derniers.

La présente Invention a pour objet une culture pure d'une souche de *Saccharomyces cerevisiae,* caractérisée en ce que ladite souche possède un ratio de croissance R, défini par la formule :

$$R = (1,5\ t_{YPG}) + t_{YNB}$$

(dans laquelle $t_{YPG}$ représente le temps de génération sur milieu riche, et $t_{YNB}$ représente le temps de génération sur milieu minimum), inférieur ou égal à 6,5, et en ce qu'elle est choisie dans le groupe constitué par :

- la souche de *Saccharomyces cerevisiae* dénommée k9, qui a été déposée le 20 Juin 1996 à la CNCM (Collection Nationale de Cultures de Microorganismes, 28, rue du Docteur Roux, 75724 PARIS, FRANCE) sous le numéro I-1736 ;
- la souche de *Saccharomyces cerevisiae* dénommée k10, qui a été déposée le 20 Juin 1996 à la CNCM sous le numéro I-1737 ;
- la souche de *Saccharomyces cerevisiae* dénommée k11, qui a été déposée le 20 Juin 1996 à la CNCM sous le numéro I-1738.
- la souche de *Saccharomyces cerevisiae* dénommée k39, qui a été déposée le 20 Juin 1996 à la CNCM sous le numéro I-1742.
- la souche de *Saccharomyces cerevisiae* dénommée k44, qui a été déposée le 20 Juin 1996 à la CNCM sous numéro 1-1743.

Cette valeur du ratio de croissance R reflète le fait que ces souches présentent à la fois un temps de génération court, c'est à dire une croissance rapide, sur milieu riche et que leur croissance n'est que peu affectée sur milieu minimum. Les Inventeurs ont constaté une corrélation entre cette caractéristique et une faible teneur du milieu en dicétones, et en particulier en diacétyle et de 2,3-pentanedione en fin de fermentation.

Selon un mode de réalisation préféré, des souches de *Saccharomyces cerevisiae* conformes à l'invention possèdent en outre les caractéristiques suivantes :

- une activité maltase importante (soit une activité maltase inductible supérieure à 0,65 micromole PNP libéré/minute/milligramme protéines totales), qui permet une utilisation précoce et rapide du maltose ;
- de bonnes propriétés de floculation, (soit une hauteur de culot de cellules supérieure à 8 millimètres, selon les conditions décrites ci-après dans l'exemple 4) ce qui évite d'avoir recours à la centrifugation pour la séparation des levures en fin de fermentation.

Les souches de levure conformes à l'invention sont utilisables pour la fabrication de la bière. La présente Invention a également pour objet cette utilisation. Dans ce cadre, les souches de l'Invention sont avantageusement utilisées isolément ou bien associées entre elles.

L'Invention a également pour objet un ferment de brasserie caractérisé en ce qu'il est constitué par une souche conforme à l'Invention, ou par un mélange de souches conformes à l'Invention.

Les levures selon l'Invention permettent un gain important sur la durée totale du procédé de fabrication de la bière et l'obtention d'un produit aux qualités organoleptiques améliorées. Le gain sur la durée du procédé de fabrication de la bière résulte à la fois d'une fermentation principale rapide et d'une réduction drastique, voire de la suppression, de la phase de maturation.

La durée de fermentation principale est réduite grâce à une vitesse de consommation des sucres fermentescibles accrue, et à une fermentation simultanée du maltose et du glucose contenus dans le moût (ces souches ne présentent pas de diauxie entraînant la consommation séquentielle du glucose et du maltose).

La phase de maturation est considérablement réduite, voire même supprimée, du fait de la production de très faibles quantités de diacétyle et de 2,3-pentanedione, et de leur résorption rapide. Ainsi la concentration de ces composés se situe à un seuil acceptable dès la fin de la phase de fermentation.

La qualité organoleptique de la bière est améliorée du fait de la rapidité des deux étapes de fermentation et de maturation, car les fermentations lentes conduisent à des produits de faible qualité.

L'utilisation des levures selon l'invention permet donc d'obtenir un gain de temps considérable sur l'ensemble du procédé de fabrication, une productivité accrue des installations de fermentation principale et une réduction d'investissement importante, sur le matériel nécessaire à la phase de maturation.

La présente Invention sera mieux comprise à l'aide du complément de description qui va suivre, qui se réfère à des exemples de mise en oeuvre des levures conformes à l'Invention.

Il doit être bien entendu toutefois que ces exemples sont donnés uniquement à titre d'illustration de l'objet de l'Invention dont ils ne constituent en aucune manière une limitation.

## EXEMPLE 1 : CARACTERISTIQUES DES SOUCHES CONFORMES A L'INVENTION

Les caractéristiques morphologiques et biolochimiques de différentes souches obtenues à partir d'un ferment industriel dénommé « mélange 2285 » sont indiquées dans les tableaux I et I bis ci-dessous.

Les souches conformes à l'invention sont les clones k9, k10, k11, k39 et k44.

TABLEAU I

| Morphologie | clone k3 | clone k9 | clone k10 | clone k11 |
|---|---|---|---|---|
| colonies | blanches, rondes, mates, bombées, régulières | blanches, rondes, mates, bombées, régulières | blanches, rondes, mates, bombées, régulières | blanches, rondes, mates, bombées, régulières |
| microscopie | grandes cellules rondes, régulières | grandes cellules rondes, régulières | grandes cellules rondes, régulières | grandes cellules rondes, régulières |
| **Fermentation** | | | | |
| Galactose | + | + | + | + |
| D-Glucose | + | + | + | + |
| D-Fructose | + | + | + | + |
| D-Mannose | + | + | + | + |
| Mannitol | - | - | - | - |
| α-méthyl-D-glucoside | - | - | - | - |
| Cellobiose | - | - | - | - |
| Maltose | + | + | + | + |
| Mélibiose | - | - | - | - |
| Saccharose | + | + | + | + |
| D-Raffinose | - | - | +( Lent) | + |
| D-Turanose | +(Lent) | - | - | + |
| **Assimilation** | | | | |
| Glucose | + | + | + | + |
| Maltose | + | + | + | + |
| Saccharose | + | + | + | + |
| Galactose | + | +(Lent) | + | + |
| Raffinose | + | + | + | + |
| Tréhalose | + (Lent) | - | + | + |
| Xylose | - | - | - | - |
| Glycérol | - | - | + (Lent) | + |
| α-méthyl-D-glucoside | - | - | - | - |
| Cellobiose | - | - | - | - |
| Mélézitose | - | - | - | - |
| Arabinose | - | - | - | - |
| **Résistance actidione** | - | - | - | - |
| **Sporulation sur CMA** | non observée | non observée | non observée | non observée |

TABLEAU I bis

| | clone k12 | clone k16 | clone k31 | clone k39 | clone k44 |
|---|---|---|---|---|---|
| **Morphologie** | | | | | |
| *colonies* | blanches, rondes, mates, bombées, régulières | blanches, rondes, mates, bombées, régulières | blanches, rondes, mates, bombées, régulières | blanches, rondes, mates, bombées, régulières | blanches, rondes, mates, bombées, régulières |
| *microscopie* | grandes cellules rondes, régulières | grandes cellules rondes, régulières | grandes cellules rondes, régulières | grandes cellules rondes, régulières | grandes cellules rondes, régulières |
| **Fermentation** | | | | | |
| Galactose | + | + | + | + | + |
| D-Glucose | + | + | + | + | + |
| D-Fructose | + | + | + | + | + |
| D-Mannose | + | + | + | + | + |
| Mannitol | - | - | - | - | - |
| α-méthyl-D-glucoside | - | - | - | - | - |
| Cellobiose | - | - | - | - | - |
| Maltose | + | + | + | + | + |
| Mélibiose | - | - | - | - | - |
| Saccharose | + | + | + | + | + |
| D-Raffinose | - | - | - | - | - |
| D-Turanose | - | - | - | - | - |
| **Assimilation** | | | | | |
| Glucose | + | + | + | + | + |
| Maltose | + | + | + | + | + |
| Saccharose | + | + | + | + | + |
| Galactose | + | + | + | + | + |
| Raffinose | + | + | + | + | + |
| Tréhalose | + | - | - | + | + |
| Xylose | - | - | - | - | - |
| Glycérol | - | - | - | - | + |
| α-méthyl-D-glucoside | - | - | - | - | - |
| Cellobiose | - | - | - | - | - |
| Mélézitose | - | - | - | - | - |
| Arabinose | - | - | - | - | - |
| **Résistance actidione** | - | - | - | - | - |
| **Sporulation sur CMA** | non observée | non observée | non observée | non observée | non observée |

## Caryotype :

On cultive les levures pendant 24 heures à 30°C sous agitation (160 rpm) sur milieu YPG (Yeast extract 2%, peptone 1%, glucose 2%).

Le caryotype de chacun des clones est analysé par électrophorèse en champs pulsés à l'aide du système CHEF MAPPER de BIO-RAD (BIO-RAD S.A., IVRY-SUR-SEINE) selon les indications du fabricant.

Deux migrations sont réalisées : l'une permettant de séparer l'ensemble des chromosomes de *Saccharomy-*

*ces cerevisiae* avec une très bonne résolution des chromosomes de poids moléculaire moyen (597 à 1005 kb) et l'autre permettant de séparer les chromosomes de petits poids moléculaire (150 à 300 kb).

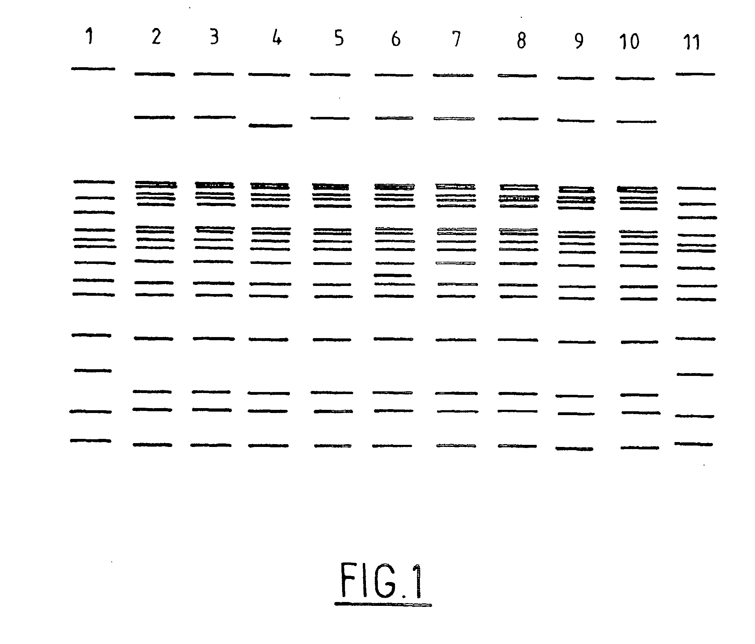

Ainsi les clones k9, k10, k11, k16, k31, k44, k39, k3 et k12 présentent les caryotypes représentés à la figure 1 :

Légende de la figure 1 (migration de l'ensemble des chromosomes)

Pistes :

1 et 11 : souche YNN295
2 : clone k11
3 : clone k9
4 : clone k10
5 : clone k16
6 : clone k31
7 : clone k44
8 : clone k39
9 : clone k3
10 : clone k12

La souche de *Saccharomyces cerevisiae* de laboratoire YNN295 a été utilisée à titre de marqueur de poids moléculaire.

## EXEMPLE 2 : TESTS DE CROISSANCE

### Milieux de cultures

Les levures sont cultivées à 30°C pendant 48 à 72 heures sur deux milieux synthétiques

Un milieu « riche » YPG constitué de :

. Extrait de levure à 2 %
. Peptone à 1 %
. Glucose à 2 %.

Un milieu « pauvre » YNB dont la composition est la suivante :

. Yeast nitrogen base (sans acides aminés) à 0,67%
. Glucose à 2 %

Les milieux solides sont obtenus en ajoutant de l'agar à 2 % aux milieux liquides.

Tous les milieux sont autoclavés 20 minutes à 110 °C.

A partir des clones cultivés sur milieu YPG ou YNB (yeast nitrogen base 0.67%, glucose 2%, agar 2%) solides, on ensemence respectivement des milieux YPG ou YNB (YNB solide sans agar) de façon à obtenir une $DO_{595\,nm} = 10^7$ cellules/ml. Chaque culture est incubée pendant 9 heures sous agitation (160 rpm) avec des prélèvements toutes les heures à partir de la mise en culture pour mesurer la $DO_{595\,nm}$. En fin d'expérience, chaque culture est analysée au microscope optique pour s'assurer de l'absence de contamination.

Le temps de génération est calculé lorsque la croissance est en phase exponentielle.

### Calcul du temps de génération (g) :

Soit X = concentration cellulaire du temps t
et n = nombre de génération

La croissance est donc du type :

$$X_{t2} = 2^n \times X_{t1}$$

d'où :

$$n = \ln(X_{t2}/X_{t1})/\ln 2$$

Le temps de génération « g » défini comme : g = t/n est calculé pour t = 2h dans la partie linéaire des courbes (comprise entre 4 h et 6 h) :

$$g = 2 \times \ln 2 / \ln(X_{t2}/X_{t1}).$$

Le temps de génération des clones k9, k10, k11, k16, k31, k44, k39, k3 et k12 est représenté dans le tableau II ci-dessous :

TABLEAU II

| Souches | Milieu pauvre (YNB) | Milieu riche (YPG) | ratio 1.5YNB+YPG |
|---|---|---|---|
| K11 | 2.50 | 1.42 | 5.17 |
| K9 | 2.65 | 2.13 | 6.10 |
| K44 | 3.25 | 1.29 | 6.16 |
| k10 | 2.80 | 2.01 | 6.21 |
| k39 | 2.90 | 2.09 | 6.44 |
| k16 | 2.95 | 2.17 | 6.69 |
| k31 | 3.50 | 1.47 | 6.72 |
| k3 | 4.43 | 2.01 | 8.65 |
| k12 | 4.29 | 2.68 | 9.07 |
| Mélange 2285 | 4.08 | 2.25 | 8.37 |

## EXEMPLE 3 : ACTIVITE MALTASE

L'activité maltase des clones est mesurée par la méthode de HALVORSON et ELIAS [Biochim. Biophys. Acta, 30, 28 (1958)].

On cultive les levures pendant 24 heures à 30°C sur milieu YPG solide. Une öse de 10 microlitres NUNC (POLYLABO) de levures est resuspendue dans 1 millilitre d'eau stérile. Les levures sont perméabilisées par addition de 0,15 millilitre de chloroforme. On agite l'ensemble pendant 30 minutes à 30°C, puis on complète à 2 millilitres avec de l'eau stérile à 4°C. La maltase constitutive est alors dosée.

L'activité maltase inductible est mesurée par culture des levures dans les mêmes conditions que ci-dessus mais sur milieu YPM solide.

L'activité maltase des clones k9, k10, k11, k16, k31, k44, k39, k3 et k12 , exprimée en micromoles de PNP (p-nitrophényl-alpha-D-glucopyranoside) libérées par minute et par milligramme de protéine totale, est représentée dans le tableau III ci-dessous :

TABLEAU III

| SOUCHES | Activité inductible (milieu YPM) | Activité constitutive (milieu YPG) |
|---|---|---|
| k12 | 0.85 | 0.20 |
| k11 | 0.81 | 0.16 |
| k3 | 0.77 | 0.16 |
| k44 | 0.67 | 0.20 |
| k9 | 0.66 | 0.16 |
| k10 | 0.66 | 0.15 |
| k39 | 0.66 | 0.15 |
| k31 | 0.67 | 0.10 |

TABLEAU III (suite)

| SOUCHES | Activité inductible (milieu YPM) | Activité constitutive (milieu YPG) |
|---|---|---|
| k16 | 0.46 | 0.11 |
| Mélange 2285 | 0.45 | 0.11 |

## EXEMPLE 4 : TEST DE FLOCULENCE

La floculence des clones est mesurée de la manière suivante. Les levures sont cultivées sur milieu YPG solide puis repiquées dans du milieu YPG à une $DO_{595\ nm}$ = 0.2 à 30°C sous agitation (160 rpm) jusqu'à épuisement du milieu. 10 millilitres de cultures sont alors transvasés dans des tubes coniques gradués (Becton Dickinson distribué par COGER, 79 rue des Morillons, 75015 PARIS CEDEX). Après 6 heures à température ambiante, la hauteur du culot de cellules floculées est mesurée en millimètre.

La floculence des clones k9, k10, k11, k16, k31, k44, k39, k3 et k12 est représentée dans le tableau IV ci-dessous :

TABLEAU IV

| SOUCHES | FLOCULENCE |
|---|---|
| k11 | B |
| k9 | B |
| k44 | B |
| k10 | B |
| k39 | B |
| k16 | B |
| k31 | B |
| k12 | B |
| k3 | M |
| Mélange 2285 | F |
| F = faible floculence, hauteur du culot de cellules inférieure à 7 millimètres<br>M = floculence moyenne, hauteur du culot de cellules comprise entre 7 et 8 millimètres<br><br>B = floculence forte, hauteur du culot de cellules supérieures à 8 millimètres. | |

## EXEMPLE 5 : TEST DES SOUCHES EN CONDITION DE FERMENTATION BRASSICOLE

Les souches conformes, ont ensuite été testées en condition de fermentation brassicole, dans des TOD (« Tank Out Door », tank cylindro conique) de 10 hectolitres. Les conditions d'entonnement sont les suivantes : moût à 14° PLATO (unité de mesure de la densité en brasserie, qui représente un pourcentage d'extrait en poids à 20°C, et correspond au poids des solutions de saccharose déterminé à 20°C, ramené au poids du même volume d'eau à 4°C.), ensemencement de $15x10^6$ cellules/ml, aération de 10 ppm pendant toute la durée de l'entonnement, température d'entonnement de 10°C. Le profil de température pour toutes les fermentations est le suivant : 10°C jusqu'à 7° PLATO, puis 12°C jusqu'à la fin de la maturation. Un suivi de densité est effectué tous les jours, un suivi des précurseurs de diacétyle et de 2,3-pentanedione est effectué jusqu'à atteinte des normes de fin de maturation (220 ppb pour le diacétyle, 180 ppb pour le 2,3-pentanedione).

Dans le tableau V suivant, les clones conformes à l'invention sont classés selon leur vitesse de fermentation, exprimée par le TPFER, durée pour atteindre 94% de l'atténuation limite (c'est-à-dire consommer 94% des sucres fermentescibles).

TABLEAU V

| CLASSEMENT | SOUCHE | TPFER |
|---|---|---|
| 1 | k11 | 158.75 |

TABLEAU V   (suite)

| CLASSEMENT | SOUCHE | TPFER |
|---|---|---|
| 2 | k9 | 159 |
| 3 | k44 | 190 |
| 4 | k39 | 204 |
| 5 | k10 | 206 |
| 6 | k16 | 215 |
| 7 | k31 | 233 |
| 8 | k12 | 257 |
| 9 | k3 | 280 |
| 10 | 2285 | 291.5 |

On observe que tous ces clones ont des TPFER inférieurs à 210 heures. Les clones sélectionnés d'après leur faible temps de génération sur milieu riche et pauvre, leur activité maltase importante et leur bonnes propriétés de floculation, à savoir les clones K11, K9, K44, K39 et K10 ont tous des TPFER inférieurs à 210 heures.

Le tableau VI ci-dessous classe les clones selon le temps mis pour résorber les précurseurs.

TABLEAU VI

| CLASSEMENT | SOUCHE | TPNOR |
|---|---|---|
| 1 | k11 | 238 |
| 2 | k12 | 255 |
| 3 | k31 | 265 |
| 4 | k39 | 280 |
| 5 | k44 | 285 |
| 6 | k9 | 295 |
| 6 | k10 | 295 |
| 6 | k16 | 295 |
| 9 | k3 | 350 |
| 9 | 2285 | 350 |

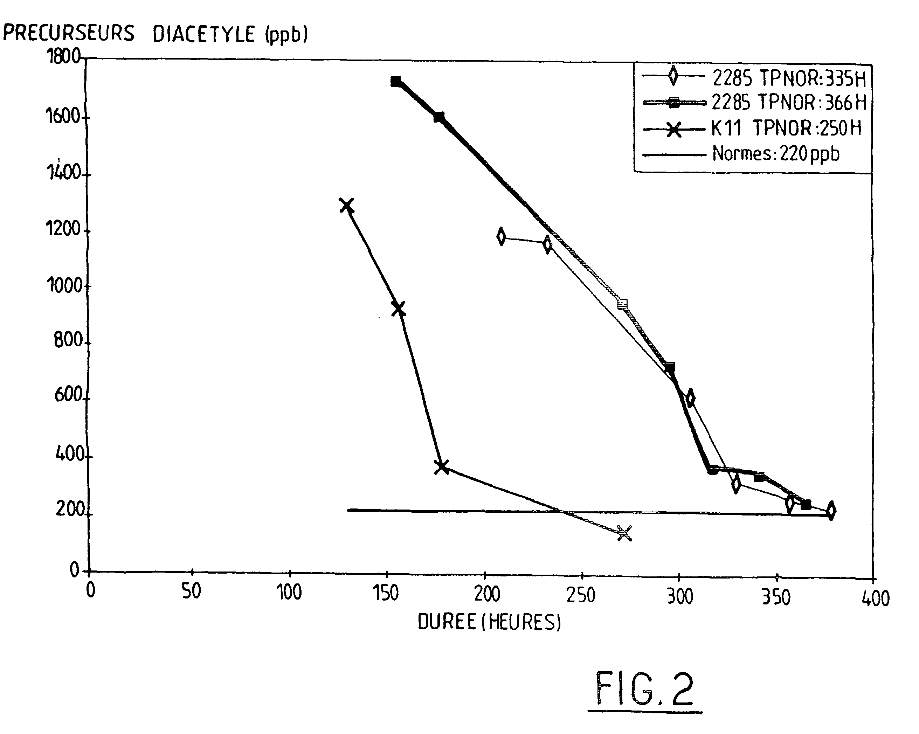

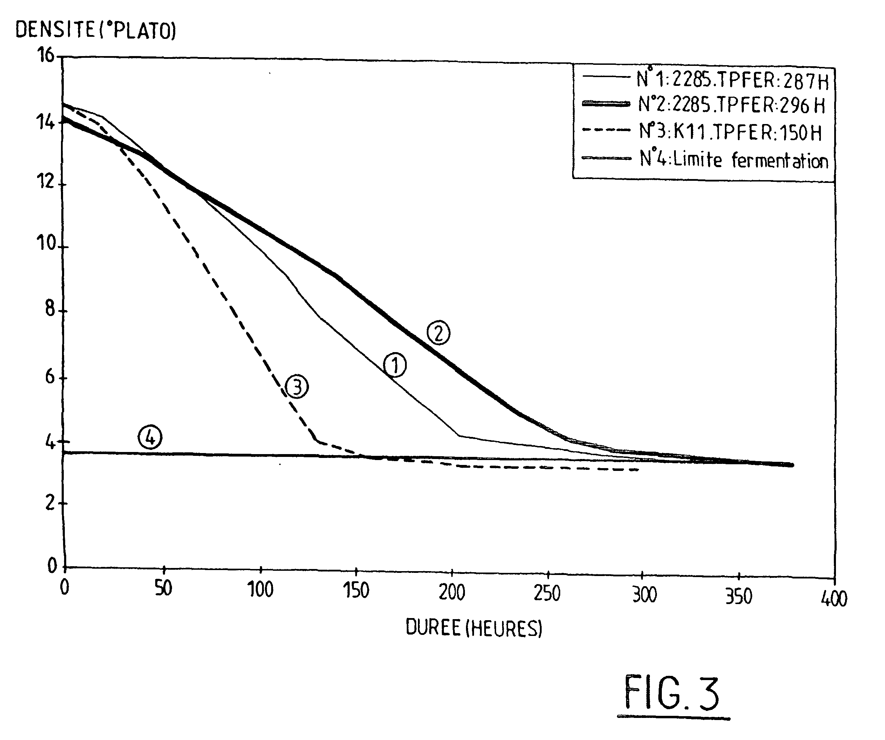

Les courbes des figures 2 et 3 montrent les comparaisons des comportements fermentaires du clone k11 et du mélange 2285. La figure 2 représente la résorption des précurseurs de diacétyle en fontion du temps de culture. La Figure 3 représente la variation de la densité en °PLATO, en fonction du temps de culture.

## Revendications

1.  Culture pure d'une souche de *Saccharomyces cerevisiae,* **caractérisée en ce que** ladite souche possède un ratio de croissance R, défini par la formule :

$$R = (1{,}5\ t_{YPG}\ ) + t_{YNB}$$

dans laquelle $t_{YPG}$ représente le temps de génération sur milieu riche, et $t_{YNB}$ représente le temps de génération sur milieu minimum,
   inférieur ou égal à 6,5, et **en ce qu'**elle est choisie dans le groupe constitué par :

-   la souche de *Saccharomyces cerevisiae* dénommée k9, qui a été déposée le 20 Juin 1996 à la CNCM (Collection Nationale de Cultures de Microorganismes, 28, rue du Docteur Roux, 75724 PARIS, FRANCE) sous le numéro I-1736 ;

- la souche de *Saccharomyces cerevisiae* dénommée k10, qui a été déposée le 20 Juin 1996 à la CNCM sous le numéro I-1737 ;
- la souche de *Saccharomyces cerevisiae* dénommée k11, qui a été déposée le 20 Juin 1996 à la CNCM sous le numéro I-1738 ;
- la souche de *Saccharomyces cerevisiae* dénommée k39, qui a été déposée le 20 Juin 1996 à la CNCM sous le numéro I-1742 ;
- la souche de *Saccharomyces cerevisiae* dénommée k44, qui a été déposée le 20 Juin 1996 à la CNCM sous le numéro I-1743.

2. Utilisation de cultures pures de souches de *Saccharomyces cerevisiae,* selon la revendication 1, pour la fabrication de la bière, lesdites souches étant utilisées isolément ou bien associées entre elles.

3. Ferment de brasserie **caractérisé en ce qu'**il est constitué par une culture pure d'une souche de *Saccharomyces cerevisiae,* ou par une culture d'un mélange de souches selon la revendication 1.

**Claims**

1. Pure culture of a strain of *Saccharomyces cerevisiae*, **characterised in that** said strain has a growth ratio R, defined by the formula:

$$R = (1.5\ t_{YPG}) + t_{YNB}$$

wherein $t_{YPG}$ represents the generation time on rich medium and $t_{YNB}$ represents the generation time on minimal medium,

of less than or equal to 6.5 and **in that** it is selected from the group consisting of:

- the strain of *Saccharomyces cerevisiae* named k9, which was deposited with the CNCM (Collection Nationale de Cultures de Microorganismes [National Collection of Microorganism Cultures], 28, rue du Docteur Roux, 75724 PARIS, FRANCE) on 20th June 1996 under number I-1736;
- the strain of *Saccharomyces cerevisiae* named k10, which was deposited with the CNCM on 20th June 1996 under number I-1737;
- the strain of *Saccharomyces cerevisiae* named k11, which was deposited with the CNCM on 20th June 1996 under number I-1738;
- the strain of *Saccharomyces cerevisiae* named k39, which was deposited with the CNCM on 20th June 1996 under number I-1742;
- the strain of *Saccharomyces cerevisiae* named k44, which was deposited with the CNCM on 20th June 1996 under number I-1743.

2. Use of pure cultures of strains of *Saccharomyces cerevisiae,* according to claim 1, in the production of beer, said strains being used individually or in combination with one another.

3. Brewer's ferment, **characterised in that** it is composed of a pure culture of a strain of *Saccharomyces cerevisiae* or of a culture of a mixture of strains according to claim 1.

**Patentansprüche**

1. Reinkultur eines *Saccharomyces cerevisiae*-Stamms, **dadurch gekennzeichnet, dass** der Stamm ein Wachstumsverhältnis R, das durch die Formel R = (1,5 $t_{YPG}$) + $t_{YNB}$, worin $t_{YPG}$ die Generationszeit auf reichhaltigem Medium darstellt und $t_{YNB}$ die Generationszeit auf Minimalmedium darstellt, definiert ist, von unter oder gleich 6,5 besitzt und dass er aus der Gruppe, bestehend aus:

- dem *Saccharomyces cerevisiae*-Stamm, der k9 genannt wird, der am 20. Juni 1996 bei der CNCM (Collection Nationale de Cultures de Microorganismes, 28, rue due Docteur Roux, 75724 PARIS, FRANKREICH) unter der Nummer I-1736 hinterlegt wurde;

- dem *Saccharomyces cersvisiae*-Stamm, der k10 genannt wird, der am 20. Juni 1996 bei der CNCM unter der Nummer 1-1737 hinterlegt wurde;

- dem *Saccharomyces cerevisiae*-Stamm, der k11 genannt wird, der am 20. Juni 1996 bei der CNCM unter der Nummer I-1738 hinterlegt wurde;

- dem *Saccharomyces cerevisiae*-Stamm, der k39 genannt wird, der am 20. Juni 1996 bei der CNCM unter der Nummer I-1742 hinterlegt wurde;

- dem *Saccharomyces* cerevisiae-Stamm, der k44 genannt wird, der am 20. Juni 1996 bei der CNCM unter der Nummer 1-1743 hinterlegt wurde, ausgewählt ist.

2. Verwendung von Reinkulturen von *Saccharomyces cerevisiae*-Stämmen nach Anspruch 1 zur Bierherstellung, wobei die Stämme isoliert oder in Kombination miteinander verwendet werden.

3. Brauferment, **dadurch gekennzeichnet, dass** es aus einer Reinkultur eines *Saccharomyces cerevisiae*-Stamms oder einer Kultur eines Gemisches von Stämmen nach Anspruch 1 besteht.

FIG.1

EP 0 818 530 B1

PRECURSEURS DIACETYLE (ppb)

Legend:
- ◇ 2285 TPNOR:335H
- ▦ 2285 TPNOR:366H
- ✕ K11 TPNOR:250H
- — Normes:220ppb

DUREE (HEURES)

<u>FIG.2</u>

EP 0 818 530 B1

FIG.3